(19) Europäisches Patentamt
European Patent Office
Office européen des brevets

(11) Publication number: **0 479 121 A1**

# (12) EUROPEAN PATENT APPLICATION

(21) Application number: **91116409.3**

(51) Int. Cl.$^5$: **A61K 7/48**

(22) Date of filing: **26.09.91**

(30) Priority: **01.10.90 IT 2161090**

(43) Date of publication of application: **08.04.92 Bulletin 92/15**

(84) Designated Contracting States: **AT BE CH DE DK ES FR GB GR IT LI LU NL SE**

(71) Applicant: **FIDIA S.p.A.**
**Via Ponte della Fabbrica 3-A**
**I-35031 Abano Terme (Padova)(IT)**

(72) Inventor: **Della Valle, Francesco**
**Via Cerato, 14**
**I-35100 Padova(IT)**
Inventor: **Romeo, Aurelio**
**Viale Ippocrate, 93**
**I-00161 Rome(IT)**
Inventor: **Rastrelli, Alessandro**
**Via Vergerio, 54**
**I-35100 Padova(IT)**

(74) Representative: **Gervasi, Gemma, Dr. et al**
**NOTARBARTOLO & GERVASI Srl 33, Viale Bianca Maria**
**I-20122 Milano(IT)**

## (54) Skin treatment compositions containing beta-phosphatidyl choline derivatives.

(57) New skin treatment compositions are described containing $\beta$-phosphatidylcholine derivatives of general formula (I):

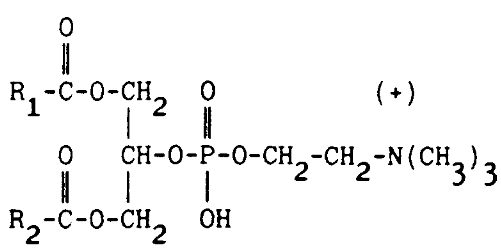

where $R_1$ and $R_2$, which can be the same or different, are pivalic acid, 3,4,5-trimethoxybenzoic acid, or $C_2$-$C_{20}$ saturated, monounsaturated or polyunsaturated linear or branched chain fatty acids, and preferably lauric, myristic, palmitic, palmitoleic, stearic, oleic, linoleic, linolenic or arachidonic acids, they possessing high emulsion stability and excellent hydrating power, and being of pleasant application.

EP 0 479 121 A1

This invention relates to skin treatment compositions containing β-phosphatidyl choline derivatives of general formula (I):

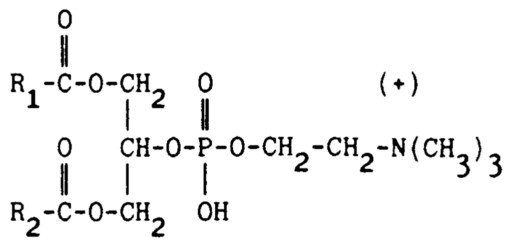

where $R_1$ and $R_2$, which can be the same or different, are pivalic acid, 3,4,5-trimethoxybenzoic acid, or $C_2$-$C_{20}$ saturated, monounsaturated or polyunsaturated linear or branched chain fatty acids, and preferably lauric, myristic, palmitic, palmitoleic, stearic, oleic, linoleic, linolenic or arachidonic acids.

Prior art

For every type of cosmetic or pharmaceutical preparation an efficient emulsion of the various ingredients is essential to ensure that the composition is stable with time and that the ingredients are uniformly distributed.

This is particularly important for emulsions comprising an aqueous phase and an oil phase, because these substances tend to separate rapidly. To stabilize the emulsion it is therefore necessary to add a third component, known as an emulsifying agent.

Synthetic emulsifying agents have been used up to the present time for this purpose and can be classified on the basis of their ionic characteristics as anionic, cationic, non-ionic or amphoteric. However, depending on the particular class, these emulsifying agents have various drawbacks such as the fact of being alkaline and hence irritant, or being incompatible with other components of the emulsion, or indeed of influencing the bioavailability of any active principles.

To prepare skin treatment compositions, lecithins (α-phosphatidyl-choline derivatives) have been used, these presenting no safety problems for the resultant compositions, but requiring particular methods for preparing the emulsion, and having to be used in combination with other surfactants.

These lecithins also have the disadvantage of generally being obtained by extraction from egg yolk or soya, together with mixtures of other phospholipids having a composition which varies considerably depending on the starting material used, these mixtures being difficult to separate into their various components. The α-lecithins obtained are in reality mixtures of α-phosphatidyl-choline derivatives, which have a very variable fatty acids composition and therefore have different characteristics depending on the fatty acids present. It is difficult to obtain α-lecithins having only the desired fatty acids.

These mixtures also contain other impurities such as proteins, inorganic substances etc., which make further purification necessary.

If unsaturated fatty acids are present, hydrogenation is required to prevent oxidation taking place.

We have now found that the use of β-phosphatidyl choline derivatives for preparing skin treatment compositions obviates the aforesaid drawbacks.

In particular, β-phosphatidyl choline derivatives are products easily prepared synthetically (in contrast to α-lecithins, β-lecithins do not comprise centres of asymmetry) and can be obtained in extremely pure form (and hence free of other phospholipids, proteins etc.) and selectively (in this respect, the desired fatty acids for the particular application can be inserted selectively, avoiding for example unsaturated acids which are subject to oxidation).

In addition to their emulsifying properties, β-phosphatidyl choline derivatives possess other properties, such as the property to hydrate, making them particularly suitable for use in preparing skin treatment compositions.

For greater clarity and brevity the following symbols will be used in the ensuing text:

$PCS_x$ = β-phosphatidyl choline derivatives
$PCS_1$ = 1,3-dipalmitoylglycero-2-phosphoryl choline
$PCS_2$ = 1,3-dimyristoylglycero-2-phosphoryl choline
$PCS_3$ = 1,3-dipivaloylglycero-2-phosphoryl choline

$PCS_4$ = 1,3-distearoylglycero-2-phosphoryl choline
$PCS_5$ = 1,3-di(3,4,5-trimethoxybenzoyl)glycero-2-phosphoryl choline

## Summary of the invention

The present invention relates to skin treatment compositions containing one or more β-phosphatidyl choline derivatives of general formula (I), an aqueous phase and an oil phase.

The skin treatment compositions of the present invention have a high degree of purity of the phospholipid composition, optimized utilization characteristics according to which fatty acids are attached to the phospholipid, a high emulsion stability and an excellent hydrating power, and are of pleasant application.

## Detailed description of the invention

The β-phosphatidyl choline derivatives of general formula (I) used according to the present invention can be prepared by one of the known methods (for example the method described in Bonsen et al.: Chemical synthesis of some lecithin analogues potential inhibitors of phospholipase A, Chem. Phys. Lipids, 8, 199-220 [1972], or J.G. Lammers, Th.F. Liefkens, J. Bus, J. Van der Meer: Syntheses and spectroscopic properties of α and β phosphatidylcholines and phosphatidylethanolamines, Chem. Phys. Lipids, 22, 293-305, [1978]).

The skin treatment compositions according to the present invention consist of an emulsion comprising one or more of the β-phosphatidyl choline derivatives of general formula (I), an oil phase, an aqueous phase and possibly components other than the aforelisted essential components.

The skin treatment compositions according to the present invention preferably consist of a quantity of between 0.05 and 30 wt% and preferably between 0.1 and 15 wt% of one or more β-phosphatidyl choline derivatives of general formula (I), a quantity of between 10 and 95 wt% and preferably between 15 and 90 wt% of aqueous phase, and a quantity of between 0.5 and 90 wt% and preferably between 1 and 85 wt% of oil phase.

The composition pH is preferably maintained between 3 and 8, in that a better emulsion stability is achieved within this range. The oil phase components used can be all those conventionally used in the cosmetics and pharmaceutical fields. Examples of substances which can be used as oil phase components are liquid or semisolid hydrocarbons such as liquid paraffin, vaseline etc; esters such as octododecyl myristate, isopropyl palmitate, cetyl-2-ethyl hexanoate, glyceryl-tri-2-ethyl hexanoate, vitamin E acetate, vitamin A palmitate, vitamin C stearate etc.; waxes such as solid paraffin, microcrystalline wax, ceresine, beeswax, spermaceti, etc.; vegetable oils and fats such as olive oil, soy oil, almond oil, etc.; animal oils and fats such as tallow, lard, mink oil, tortoise oil, etc; higher alcohols such as cetyl, stearyl, cetostearyl, oleic alcohol etc.; higher fatty acids such as lauric, myristic, palmitic, stearic, oleic, linoleic, linolenic, isostearic acid etc.; silicone oils such as dimethyl silicone, methylphenyl silicone etc. These substances can be used individually or in mixture.

Aqueous phase components which can be used in the present invention include water, monovalent alcohols such as ethanol, propanol, isopropanol, butanol etc.; polyvalent alcohols with two or more hydroxyl groups such as ethylene glycol, propylene glycol, 1,3-butylene glycol, 1,4-butylene glycol, glycerin, glucose, maltose, saccharose, sorbitol, etc.; and other aqueous phase components which are normally used in the production of cosmetics, pharmaceutical products etc. These substances can be used individually or in mixture.

The emulsion of the present invention can also contain components other than the aforestated essential components, for example various additives such as pharmaceutical substances, UV absorbents, preservatives, sterilizers, antioxidants, perfumes, dyes, thickeners. stabilizers etc. In addition to the currently used non-ionic, anionic, cationic and amphoteric surfactants, natural surfactants can be used such as casein, sodium caseinate, saponin etc. according to the use for which the skin treatment product is intended.

The compositions of the present invention can be prepared by emulsifying the aforestated components using conventional emulsifying systems such as homogenizers etc.

In order to demonstrate the characteristics and advantages of the skin treatment compositions of the present invention, certain tests were conducted and are described below.

TEST 1

## Emulsifying power of β-phosphatidyl choline derivatives

20 mg of each synthetic lecithin were added to 1 g of a 1:1 w/w $H_2O$/isopropyl myristate and the mixtures, in graduated test-tubes, were agitated at 40 °C for 15 minutes.

The mixtures were returned to ambient temperature and the emulsive power was evaluated by measuring, 30 minutes after agitation, the height of the entire solution A compared with the height of the emulsified phase B.

The ratio B/A x 100 gives a measure of the emulsive power, with a maximum of 100% and a minimum of 0%, of the β-phosphatidyl choline derivatives considered.

The results are given in Table 1.

TABLE 1

| β-phosphatidyl choline derivative | B/A x 100 |
|---|---|
| $PCS_2$ | 100% |
| $PCS_4$ | 100% |

From the results given in the table it can be deduced that $PCS_2$ and $PCS_4$ have maximum emulsifying power under the conditions considered.

TEST 2

Evaluation of the influence of β-phosphatidyl choline derivatives on the stability of a standard emulsion

To evaluate the influence of β-phosphatidyl choline derivatives on emulsion stability, a standard emulsion was prepared having the following composition (Table 2):

TABLE 2

| Component | % by weight |
|---|---|
| Oil phase: | |
| Cremophor $A_6$ | 2.5 |
| Cremophor $A_{25}$ | 1.5 |
| Isopropyl myristate | 5.0 |
| Almond oil | 2.0 |
| Cetiol V | 5.0 |
| Cetyl alcohol | 5.0 |
| Aqueous phase: | |
| Glycerol | 1.0 |
| Water | 78.0 |

The aqueous phase heated to 70 °C is slowly added to the oil phase heated to 75 °C. Rapid agitation is effected by a Silverson laboratory mixer equipped with an emulsifying head and is continued for about 30 minutes, slowly cooling the formed emulsion to ambient temperature.

250 mg of each of the considered β-lecithins dispersed or dissolved in 10 g of water are added under slow agitation to 90 g of the standard emulsion obtained.

The resultant emulsions are tested for stability by subjecting them to centrifuging at 6000 r.p.m. for 10, 30 and 60 minutes respectively, and by storing them under temperature control at 45 °C for 72 hours. In

both cases the stability is evaluated by noting any phase separation.

The results are given in Tables 3 and 4.

TABLE 3

Stability under centrifuging at 6000 r.p.m. for 10', 30' and 60'

| Emulsion | 10 min | 30 min | 60 min |
|---|---|---|---|
| Standard emulsion + $PCS_1$ | Stable | Stable | Stable |
| " " + $PCS_2$ | " | " | " |
| " " + $PCS_3$ | " | " | " |
| " " + $PCS_4$ | " | " | " |

TABLE 4

Stability under temperature control at 45°C for 72 h.

| Emulsion | Stability |
|---|---|
| Standard emulsion | Stable |
| " " + $PCS_1$ | " |
| " " + $PCS_2$ | " |
| " " + $PCS_3$ | " |
| " " + $PCS_4$ | " |
| " " + $PCS_5$ | " |

The results show that the β-phosphatidyl choline derivatives have no negative influence on the stability of the tested emulsions.

TEST 3

In vivo hydration tests on human volunteers

The hydration tests were conducted using a Corneometer laboratory instrument equipped with a capacitive probe able to measure the moisture present on the stratum corneum. To conduct the test, 50 mg of standard cream and cream containing the various synthetic β-lecithins prepared as Examples 1 and 2 are applied to the volar surface of the forearm (20 $cm^2$ ) with light circular massage.

The hydration measurements were taken before applying the product and 30 min, 150 min and 270 min after its application. The hydration values are expressed in arbitrary units deriving from electrical impedance measurements on the cutaneous surface. Although the values are relative they are in inverse linear relationship to the absolute water quantity present in the cutaneous layer of the skin (lower numbers correspond to higher hydration).

The results, expressed as the average of the data obtained for 10 subjects, are given in Table 5.

TABLE 5

| Emulsion | Measured hydration values | | | |
|---|---|---|---|---|
| | Before application | 30' | 150' | 270' |
| Standard emulsion | 241±21 | 168±15 | 184±14 | 230±20 |
| Emulsion + $PCS_1$ | 241±21 | 165±10 | 195±15 | 213±15 |
| " + $PCS_2$ | 241±21 | 147±11 | 175±10 | 195±9 |
| " + $PCS_3$ | 241±21 | 157±7 | 193±13 | 206±9 |
| " + $PCS_4$ | 241±21 | 132±12 | 161±11 | 180±12 |
| " + $PCS_5$ | 241±21 | 124±14 | 144±16 | 177±15 |

The measured hydration data and in particular the values referring to 270 minutes after its application show that the emulsions containing $PCS_x$, and particularly those containing $PCS_2$, $PCS_4$ and $PCS_5$, exercise a considerable hydrating effect on the skin. This effect remains even after a lengthy period.

Two non-limiting examples of compositions formed in accordance with the present invention are given below.

EXAMPLE 1

Preparation of standard emulsions of hydrophilic ointment type containing $PCS_x$

A hydrophilic ointment was prepared starting from the base described under the item "Cetomacrogel Emulsifying Base" of the British Pharmacopea 1988.

The composition of the final cream is in particular given in Table 6.

TABLE 6

| Component | | % by weight |
|---|---|---|
| Cetomacrogel 1000 | | 1.80 |
| Cetyl alcohol | | 7.20 |
| White vaseline | | 15.00 |
| Liquid paraffin | | 6.00 |
| $PCS_1$ | | 2.00 |
| Methyl Paraben | | 0.20 |
| Propyl Paraben | | 0.02 |
| Water | to make up to | 100.00 |

These preparations containing $PCS_1$ are slightly oily brilliant white creams of pleasing sensation on application.

Emulsions containing $PCS_2$, $PCS_3$, $PCS_4$, and $PCS_5$ were prepared in exactly the same manner.

The products were stable both under the centrifuge test and under the temperature-controlled storage test.

EXAMPLE 2

Preparation of fluid emulsions containing $PCS_x$

A fluid emulsion containing $PCS_1$ was prepared using the formulation of Table 7

TABLE 7

| | Component | % by weight |
|---|---|---|
| Oil phase: | Glucate DO | 5.00 |
| | Elfacos ST9 | 3.00 |
| | Squalane | 30.00 |
| | $PCS_1$ | 2.00 |
| Aqueous phase: | Glycerol | 2.00 |
| | Methyl Paraben | 0.20 |
| | Propyl Paraben | 0.02 |
| | Water to make up to | 100.00 |

The aqueous phase heated to 70 ° C is slowly added to the oil phase heated to 75 ° C.

Rapid agitation is effected by a Silverson laboratory mixer equipped with an emulsifying head and is continued for about 30 minutes, slowly cooling the formed emulsion to ambient temperature. The final emulsions containing the $PCS_x$ β-lecithins were fluid milks of white A/O type which were slightly oily, runnable and of pleasant application.

Emulsions containing $PCS_2$, $PCS_3$, $PCS_4$, and $PCS_5$ were prepared in exactly the same manner.

The products were stable both under the centrifuge test and under the temperature-controlled storage test.

## Claims

1. Skin treatment compositions containing an aqueous phase, an oil phase and one or more β-phosphatidyl choline derivatives of general formula (I):

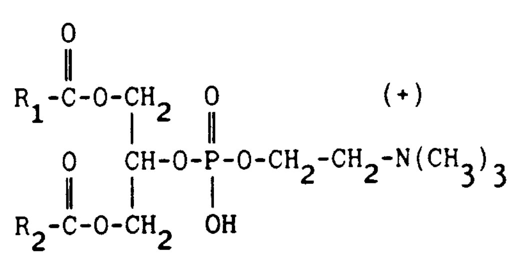

where $R_1$ and $R_2$, which can be the same or different, are chosen from the group consisting of pivalic acid, 3,4,5-trimethoxybenzoic acid, and $c_2$-$c_{20}$ saturated, monounsaturated or polyunsaturated linear or

branched chain fatty acids.

2. Skin treatment compositions as claimed in claim 1, characterised in that $R_1$ and $R_2$ are the same and are myristic or stearic acid.

3. Skin treatment compositions as claimed in claim 1, characterised by consisting of a quantity of between 0.05 and 30 wt% of one or more $\beta$-phosphatidyl choline derivatives of general formula (I), a quantity of between 10 and 95 wt% of aqueous phase, and a quantity of between 0.5 and 90 wt% of oil phase.

4. Skin treatment compositions as claimed in claim 3, characterised by consisting of a quantity of between 0.01 and 15 wt% of one or more $\beta$-phosphatidyl choline derivatives of general formula (I), a quantity of between 15 and 90 wt% of aqueous phase, and a quantity of between 1 and 85 wt% of oil phase.

5. The use of $\beta$-phosphatidyl choline derivatives of general formula (I) for preparing skin treatment compositions.

**Claims for the following Contracting States : ES, GR**

1. Process for the preparation of skin treatment compositions containing as active principle an active dosis of one or more $\beta$-phosphatidyl choline derivatives of formula (I)

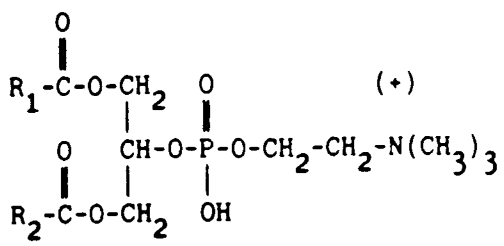

wherein $R_1$ and $R_2$, which are same or different, are chosen in the group of pivalic acid, 3,4,5-trimethoxybenzoic acid and $C_2$-$C_{20}$ satured, monounsatured or polyunsatured linear or branched chain fatty acids, an aqueous and an oil phase and comprising the mixture of said active principle with the above said phases.

2. Process for the preparation of skin treatment compositions according to claim 1, wherein $R_1$ and $R_2$ are the same and are myristic or stearic acid.

3. Process for the preparation of skin treatment compositions according to claim 1, wherein the compositions consist of a quantity comprised between 0.05 and 30 wt% of one or more of the choline derivatives of formula (I), a quantity comprised between 10 and 95 wt% of aqueous phase and a quantity comprised between 0.5 and 90 wt% of oil phase.

4. Process for the preparation of skin treatment compositions according to claim 3, wherein the compositions consist of a quantity comprised between 0.1 and 15 wt% of one or more $\beta$-phosphatidylcholine of formula (I), a quantity comprised between 15 and 90 wt% of aqueous phase and a quantity comprised between 1 and 85 wt% of oil phase.

5. Use of the $\beta$-phosphatidyl choline derivatives of formula (I) for the preparation of skin treatment compositions.

European Patent Office

# EUROPEAN SEARCH REPORT

Application Number

EP 91116409.3

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| A<br><br><br><br>X | WO - A - 90/06 103<br>(THE BOOTS CO)<br>* Claims; page 3, lines 3-21 *<br>-- | 1-4<br><br><br><br>5 | A 61 K 7/48 |
| Y | EP - A - 0 256 821<br>(ADVANCED ORAL HEALTH CORP.)<br>* Claims; page 2, line 46 - page 3, line 10, examples 8,14 *<br>-- | 1-5 | |
| Y | HANDBUCH DER LEBENSMITTEL-CHEMIE, vol. 1, 1965, Springer-Verlag, Berlin, Heidelberg, New York;<br>J. SCHORMÜLLER "Die Bestandteile der Lebensmittel"<br>pages 1173,1174<br>* Page 1174 *<br>-- | 1-5 | TECHNICAL FIELDS SEARCHED (Int. Cl.5) |
| A | US - A - 4 525 344<br>(R. J. TUTSKY)<br>* Claims *<br>---- | 1-5 | A 61 K 7/00 |

The present search report has been drawn up for all claims

| Place of search | Date of completion of the search | Examiner |
|---|---|---|
| VIENNA | 09-12-1991 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)